# EUROPÄISCHE PATENTSCHRIFT

(11) **EP 1 036 791 B1**
(45) Veröffentlichungstag und Bekanntmachung des Hinweises auf die Patenterteilung: **16.01.2002**
(21) Anmeldenummer: 00103297.8
(22) Anmeldetag: 18.02.2000
(51) Int. Cl.: C07D 309/30, C07D 315/00

(54) **Verfahren zur Herstellung von Delta-Lacton**
Process for the preparation of delta-lactons
Procédé pour la préparation de delta-lactones

(30) Priorität: 16.03.1999 DE 19911608
(43) Veröffentlichungstag der Anmeldung: 20.09.2000
(73) Patentinhaber: Forschungszentrum Karlsruhe GmbH, 76133 Karlsruhe (DE)
(72) Erfinder: Gassner, Franz, Dr., 76351 Linkenheim-Hochstetten (DE); Haack, Vera, Dr., 07743 Jena (DE); Janssen, Annette, 76297 Stutensee (DE); Elsagir, Anja, Dr., 57076 siegen (DE); Dinjus, Eckhard, Prof. Dr., 76774 Leimersheim (DE)

(56) Entgegenhaltungen:
- DE-A- 19 725 735
- GB-A- 2 003 875

## Beschreibung

Die Erfindung betrifft ein Verfahren zur Herstellung von δ-Lacton gemäß dem Oberbegriff des ersten Patentanspruchs.

Ein solches Verfahren ist aus der DE 197 25 735 A1 bekannt. Butadien und Kohlendioxid werden mit einem Palladium-Katalysator zu δ-Lacton umgesetzt, wobei ein Kohlendioxid-Druck von 5 bis 100 bar, vorzugsweise zwischen 20 und 30 bar, verwendet wird. Der Palladium-Katalysator wird durch Umsetzung eines phosphinoalkyl-funktionalisierten Polystyrols mit (η⁵-cyclopentadienyl) (η³-allyl)palladium gewonnen.

Der Erfindung liegt die Aufgabe zugrunde, ein Verfahren der eingangs genannten Art vorzuschlagen, das höhere Ausbeuten bietet und die Verwendung einfacherer Palladium-Katalysatoren ermöglicht.

Die Aufgabe wird erfindungsgemäß durch das im ersten Patentanspruch gekennzeichnete Merkmal gelöst. Die weiteren Ansprüche geben bevorzugte Ausführungsformen des Verfahrens an.

Erfindungsgemäß werden Butadien, der Katalysator und Kohlendioxid in einem Druckbehälter vorgelegt. Das Butadien/Kohlendioxid-Molverhältnis wird bevorzugt auf 1,1 bis 1,5 eingestellt. Im Unterschied zu der oben zitierten DE 197 25 735 A1 wird der Druck jedoch nicht dadurch aufgebracht, daß Kohlendioxid aufgepreßt wird, sondern in der Weise, daß sich das einmal eingestellte Molverhältnis Butadien/Kohlendioxid nicht mehr ändert. Der Druck kann beispielsweise durch Aufpressen eines Edelgases wie z. B. Argon oder durch Einsatz einer Vorrichtung, mit der das Volumen der Ausgangskomponenten vermindert wird, etwa einer Spindelpresse, erzeugt werden. Der erzeugte Druck sollte dabei so hoch wie technisch möglich eingestellt werden und mindestens 500 bar bis 7000 bar betragen, wobei Drücke über 1000 bar bevorzugt werden.

Die Erfindung beruht auf der Beobachtung, daß ein hoher Kohlendioxid-Partialdruck den Palladium-Katalysator inaktiviert, wobei der ungünstige Einfluß des hohen Kohlendioxid-Partialdrucks auf den Katalysator die infolge des Massenwirkungsgesetzes zu erwartende Ausbeuteerhöhung bei weitem überkompensiert.

Ein entscheidender Vorteil der Erfindung besteht darin, daß bei der vorgeschlagenen Art der Druckerzeugung ein wesentlich einfacherer Palladium-Katalysator eingesetzt werden kann. Der Katalysator braucht nicht phosphinoalkyl-funktionalisiert sein, sondern es eignen sich wesentlich einfacher aufgebaute organometallische Palladium(0)- und Palladium(II) -Verbindungen (mit PPh₃ als einfachem Phosphan) wie beispielsweise Pd₂dba₃ (dba = Dibenzylidenaceton) oder Pd(OAc)₂ (OAc = Acetat) und Pd(acac)₂ (acac = Acetylacetonat). Damit vereinfacht sich die δ-Lacton-Synthese erheblich. Ein weiterer Vorteil ist, daß die Reaktion bei ca. 40°C und somit bei erheblich niedrigerer Temperatur als beim bekannten Verfahren durchgeführt werden kann.

Die Erfindung wird im folgenden anhand eines Ausführungsbeispiels näher erläutert.

Eine Reaktionslösung aus 20 ml Acetonitril, 110,9 mg (0,121 mmol) Katalysator Pd₂dba₃, 10,65 g (197 mol) Butadien und 11,26 g (0,256 mol) CO₂ wurde in einem Druckbehälter bei Raumtemperatur und unter dem maximalen Dampfdruck der jeweiligen Komponenten vorgelegt. Der für die Reaktion erforderliche Systemdruck wird dadurch erzeugt, daß die flüssige Phase der Reaktionsmischung bei Raumtemperatur aus dem Druckbehälter in einen Reaktor überführt und im Reaktor mechanisch mittels einer Handspindelpresse ein Druck von ca. 1000 bar erzeugt wurde. Der Reaktionsautoklav wird beheizt. Nach einer Reaktionszeit von 18 h und einer Reaktionstemperatur von max. 80°C wurde die Heizung abgestellt und der Reaktorinhalt in einen zweiten Druckbehälter, der ein im Verhältnis zum Reaktorvolumen wesentlich größeres Volumen aufweist, überführt. Es ergab sich eine Ausbeute (GC) von 248,3 mg/g δ-Lacton. Ein bei 35 bar durchgeführter Vergleichsversuch ergab unter sonst identischen Bedingungen 20,3 mg/g δ-Lacton.

## Patentansprüche

1. Verfahren zur Herstellung von δ-Lacton, bei dem man Butadien mit Kohlendioxid unter Druck in Gegenwart eines Katalysators umsetzt,
**dadurch gekennzeichnet, daß**
Butadien und Kohlendioxid vorgelegt und der Druck in der Weise erzeugt wird, daß sich das Molverhältnis Butadien/Kohlendioxid nicht ändert.

2. Verfahren nach Anspruch 1,
**dadurch gekennzeichnet, daß**.
Kohlendioxid in einem 1,1- bis 1,5-fachen stöchiometrischen Überschuß vorgelegt wird.

3. Verfahren nach Anspruch 1,
**dadurch gekennzeichnet, daß**
der Druck mittels einer Spindelpresse erzeugt wird.

4. Verfahren nach Anspruch 1,
**dadurch gekennzeichnet, daß**
der Druck durch Aufpressen eines Inertgases erzeugt wird.

5. Verfahren nach Anspruch 3 oder 4,
**dadurch gekennzeichnet, daß**
der Druck 500 bar bis 7000 bar beträgt.

6. Verfahren nach einem der Ansprüche 1 bis 5,
**dadurch gekennzeichnet, daß**
als Katalysator Pd₂dba₃ mit dba = Dibenzylidenaceton eingesetzt wird.

7. Verfahren nach einem der Ansprüche 1 bis 5,
**dadurch gekennzeichnet, daß**
als Katalysator Pd(PPh₃)₄ oder Pd(OAc)₂/PPh₃ mit Ac = Acetat eingesetzt wird.

## Claims

1. Method of producing δ-lactone, wherein butadiene is reacted with carbon dioxide under pressure in the presence of a catalyst, **characterised in that** butadiene and carbon dioxide are provided, and the pressure is produced in such a manner that the molar ratio of butadiene : carbon dioxide does not change.

2. Method according to claim 1, **characterised in that** carbon dioxide is provided in 1.1- to 1.5-times a stoichiometric excess.

3. Method according to claim 1, **characterised in that** the pressure is produced by means of a spindle press.

4. Method according to claim 1, **characterised in that** the pressure is produced by compressing an inert gas.

5. Method according to claim 3 or 4, **characterised in that** the pressure is 500 bar to 7000 bar.

6. Method according to one of claims 1 to 5, **characterised in that** Pd₂dba₃, with dba = dibenzylidene acetone, is used as the catalyst.

7. Method according to one of claims 1 to 5, **characterised in that** Pd(PPh₃)₄ or Pd(OAc)₂/PPh₃, with Ac = acetate, is used as the catalyst.

## Revendications

1. Procédé de préparation de delta-lactone, dans lequel on fait réagir du butadiène avec de l'anhydride carbonique sous pression en présence d'un catalyseur,
**caractérisé en ce qu'**
on dispose le butadiène et l'anhydride carbonique et on produit la pression de manière que le rapport molaire du butadiène à l'anhydride carbonique ne se modifie pas.

2. Procédé selon la revendication 1,
**caractérisé en ce qu'**
on dispose l'anhydride carbonique en un excès stoechiométrique d'un facteur de 1,1 à 1,5.

3. Procédé selon la revendication 1,
**caractérisé en ce qu'**
on produit la pression au moyen d'une presse à vis.

4. Procédé selon la revendication 1,
**caractérisé en ce qu'**
on produit la pression en comprimant un gaz inerte.

5. Procédé selon la revendication 3 ou 4,
**caractérisé en ce que**
la pression est de 500 bars à 7000 bars.

6. Procédé selon l'une des revendications 1 à 5,
**caractérisé en ce qu'**
on utilise comme catalyseur Pd₂dba₃ avec dba = dibenzylidène acétone.

7. Procédé selon l'une des revendications 1 à 5,
**caractérisé en ce qu'**
on utilise comme catalyseur Pd(PPh₃)₄ ou Pd(OAc)₂/PPh₃ avec Ac = acétate.
